# EUROPEAN PATENT SPECIFICATION

(11) **EP 2 955 650 B1**
(45) Date of publication and mention of the grant of the patent: **15.05.2019**
(21) Application number: 15170676.9
(22) Date of filing: 04.06.2015
(51) Int. Cl.: G16H 20/30, G16H 40/63

(54) **PORTABLE APPARATUS FOR TRANSFERRING DATA USING PROXIMITY WIRELESS CONNECTION**
TRAGBARE VORRICHTUNG ZUR ÜBERTRAGUNG VON DATEN MITTELS DRAHTLOSER NÄHERUNGSVERBINDUNG
APPAREIL PORTATIF PERMETTANT DE TRANSFÉRER DES DONNÉES AU MOYEN D'UNE CONNEXION SANS FIL DE PROXIMITÉ

(30) Priority: 12.06.2014 US 201414303044
(43) Date of publication of application: 16.12.2015
(73) Proprietor: Polar Electro Oy, 90440 Kempele (FI)
(72) Inventor: Tilvis, Marko, 90440 Kempele (FI); Erkkilä, Mika, 90440 Kempele (FI)
(74) Representative: Kolster Oy Ab

(56) References cited:
- EP-A1- 2 239 023
- US-A1- 2007 219 059
- US-A1- 2011 152 695
- US-A1- 2014 039 337
- US-A1- 2015 015 502
- US-B1- 8 725 842

## Description

### FIELD OF THE INVENTION

The invention relates generally to transferring data from a portable training apparatus to another apparatus. More particularly, the present invention relates to using proximity wireless connection for the data transfer.

### BACKGROUND OF THE INVENTION

The user interface of smart watches and wrist-worn training computers is often limited due to small display size. One example may be a viewing of a physical activity file or a user editable configuration template. The viewing can be easier and more efficient to perform on an apparatus with a larger display. Another aspect is that it is easier for a user to edit information of the user editable configuration template on a larger display.

US8725842 discloses a smart watch is including a band unit, a network unit, a display unit and a control unit. The band unit is configured to be worn on a user's wrist. The smart watch includes a band unit that is capable of being worn on a user and includes a wrist band layer disposed on the band unit, an image capture device disposed on the band unit, a power source coupled to the image capture device, a memory coupled to the image capture device, and a motion detector coupled to the memory. The smart watch includes a network unit located in the band unit and communicates wirelessly with at least one of a portable network device and a server and a display unit being coupled to the power source, and a control unit that includes a processor and coupled to the memory, the power source, the display unit, and the network unit, the control unit receiving information from the image capture device, and the motion detector and storing information in the memory.

EP2239023 discloses an interaction between a portable apparatus and a personal exercise area. A disclosed method comprises: transferring wirelessly information between a personal exercise area and a portable apparatus; detecting proximity of the portable apparatus to the personal exercise area by utilizing the transferred information; and configuring the portable apparatus in relation to an exercise performed within the personal exercise area by a user of the portable apparatus.

US2011/152695 discloses a method, apparatuses, and a system for carrying out exercise-related processing. The apparatus includes an exercise-measurement circuitry configured to measure exercise-related measurement data related to a user carrying out an exercise. The apparatus further comprises a communication circuitry configured to provide the portable apparatus with wireless communication capability. The apparatus further comprises a processing circuitry configured to receive the exercise-related measurement data from the exercise-measurement circuitry, to process the exercise-related measurement data, and to communicate with an external user interface apparatus over a bidirectional wireless communication connection through the communication circuitry so as to cause transmission of the processed exercise-related measurement data to the user interface apparatus and to receive configuration data from the user interface apparatus.

US2011/152695 discloses a method and system for continuous monitoring, real-time analysis, and automated and personalized training of exercise. The system embodies a multi-sensor data acquisition system to measure body sounds, body signs, vital signs, motions, and machine settings continuously and automatically. The system is able to capture the body sounds and other vital signs, analyze them, and report and display summarized results. The signal processing functions utilize a unique signal separation and noise removal methodology by which authentic signals can be extracted from interfered signals and in noisy environments, even when signals and noises have similar frequency components or are statistically dependent.

### BRIEF DESCRIPTION OF THE INVENTION

According to an aspect of the invention, there is provided the portable apparatus of claim 1.

In an example, the processing circuitry is further configured to generate a physical activity file comprising physical activity-related measurement data, and wherein the communicated reference data is associated with the physical activity file. In an embodiment, the communicated reference data dynamically changes according to the physical activity file that is currently selected.

In an example, the proximity wireless connection circuitry is further configured to transfer the physical activity file from the portable apparatus to a training database.

In an example, the communicated reference data, by the portable apparatus, causes the second apparatus to fetch and illustrate the physical activity file.

In an example, the proximity wireless connection circuitry is configured to automatically change the communicated reference data to correspond the physical activity file viewed by the user.

According to an aspect of the invention, there is provided the computer program product of claim 12.

According to an aspect of the invention, there is provided the system of claim 13.

Further embodiments are defined in the dependent claims.

### BRIEF DESCRIPTION OF THE DRAWINGS

Embodiments of the present invention are described below, by way of example only, with reference to the accompanying drawings, in which
Figure 1A illustrates an exemplary system for measuring and displaying physical activity-related data in order to monitor a physical activity performed by a user;
Figure 1B illustrates the using of the proximity wireless connection;
Figure 2 illustrates the selecting of data to be communicated via the proximity wireless connection;
Figure 3 illustrates a flow diagram according to an embodiment of the invention;
Figure 4 illustrates a user editable configuration template information communication;
Figure 5 illustrates communication of a reference data according to an embodiment of the invention;
Figure 6 illustrates a user editable configuration template transfer according to an embodiment of the invention;
Figure 7 illustrates a block diagram of an apparatus according to an embodiment of the invention; and
Figure 8 illustrates a block diagram of an apparatus according to an embodiment of the invention.

### DETAILED DESCRIPTION OF THE INVENTION

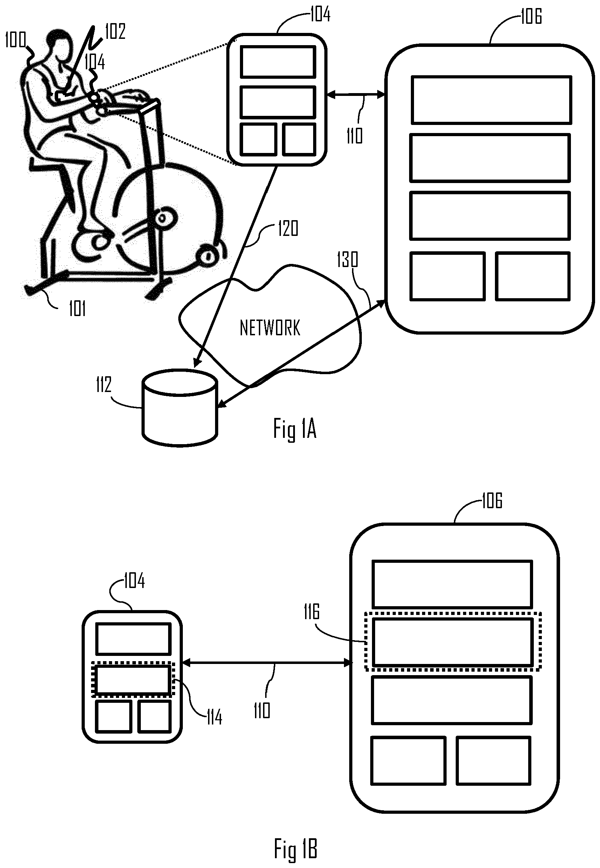

Figure 1A illustrates an exemplary system for measuring and displaying physical activity-related data in order to monitor a physical activity performed by a user 100. Referring to Figure 1A, the user 100 may have various sensor devices 102, such as heart activity sensors, that measure and process the physical activity-related data. In an embodiment, at least one of the various sensor devices 102 is a heart activity sensor comprising at least one electrical or optical sensor to measure heart activity of the user 100. Other types of physical activity sensors may also be applied by the user 100. These may include, e.g., a cadence sensor and a power sensor, to mention only a few. The user 100 may wear a portable apparatus 104 which is capable of receiving the physical activity-related data, from the various sensor devices 102, and displaying at least some of the contents of the physical activity-related data to user 100. The portable apparatus 104 may further process at least some of the physical activity-related data and display the processed contents to the user. From the portable apparatus 104, the user 100 may monitor physical activity-related parameters that characterize user's physiological state. This may be detected from the physical activity-related data, such as heart rate information, e.g. by monitoring how the heart rate changes as the physical activity session goes on. In an embodiment, at least some of the physical activity-related data may further be transferred 120 from the portable apparatus 104 to a training database 112 which may be accessed via network.

Various sensor devices 102 are comprised in the portable apparatus 104.

However, monitoring training parameters from the relatively small portable apparatus 104 may not be easy. Similarly, it may be troublesome to edit portable apparatus configuration from a small device. Referring to Figure 1A, the portable apparatus 104 may be connected to a second apparatus 106 by a proximity wireless connection 110. The proximity wireless connection 110 is used to transfer data from the portable apparatus 104 to the second apparatus 106 or vice versa. The portable apparatus 104 communicates reference data, using the proximity wireless connection 110, to the second apparatus 106 when the second apparatus 106 is in proximity of the portable apparatus 104.

The reference data may comprise, for example, an identifier, which is associated with portable apparatus 104 -related information. The portable apparatus 104 -related information may be, for example, the physical activity-related data stored from the various sensor devices 102 or from the portable apparatus 104 to the training database 112. In an embodiment, the portable apparatus 104 -related information comprises information which specifies the portable apparatus, i.e. apparatus model, software version, user parameters and user information, for example. The information specifying the portable apparatus may be a unique identifier, for example.

The identifier, transmitted from the portable apparatus 104 to the second apparatus 106, may be used, by the second apparatus 106, to fetch 130 information associated with the identifier from the training database 112. The fetched information may be further displayed on the second apparatus 106 providing a larger display. The fetched information is viewed on the second apparatus 106, edited and at least some of the information is transferred from the second apparatus 106 to the portable apparatus 104 using the proximity wireless connection 110.

In an embodiment, reference data associated with the data provided by the various sensor devices 102 is communicated to the second apparatus 106 by the portable apparatus 104 using the proximity wireless connection 110. The data provided by the various sensor devices 102 may be then fetched using the communicated reference, and further displayed and edited on the second apparatus 106. At least some of the edited data provided by the various sensor devices 102 may be communicated from the second apparatus 106 to the portable apparatus 104 using the proximity wireless connection 110.

Figure 1B illustrates the using of the proximity wireless connection 110. Referring to Figure 1B, proximity wireless connection setting 114 may be enabled for the portable apparatus 104 by setting it on from the user interface of the portable apparatus 104. Similarly, the proximity wireless connection setting 116 may be enabled for the second apparatus 106 by setting it on from the user interface of the second apparatus 106. In an embodiment, the proximity wireless connection 110 automatically turns on when a physical activity-related measurement data or the processed data is viewed by the user on the portable apparatus 104. By enabling both proximity wireless settings 114, 116, proximity wireless connection 110 between apparatuses 104, 106 may be made possible. Proximity wireless connection 110 may transfer information, as described earlier, from apparatus to another when the apparatuses are in proximity of each other. When the proximity wireless connection 110 is made possible, the portable apparatus 104 detects that the second apparatus 106 is in proximity of it and communicate reference data, comprising an identifier associated with information, to the second apparatus. The reference data causes the second apparatus to automatically fetch the information associated with the identifier from a data source, such as a training database.

In an embodiment, the proximity wireless connection 110 applies one of the following short range device-to-device communication technologies: Near Field Communication (NFC), Radio Frequency Identification (RFID), Bluetooth, Bluetooth Low Energy, wireless local area network (WLAN), ANT or ANT+ by Dynastream, or IEEE 802.15.4. Other short-range device-to-device or network communication protocols are equally possible. The detection of proximity using previously mentioned short-range device-to-device communication technologies may utilize, for example: NFC, received signal strength indication (RSSI) or iBeacon by Apple. Other proximity detection protocols or technologies are equally possible. The NFC is a technique enabling radio frequency identification in very short distances.

In an embodiment, the range of the proximity wireless connection 110 is between 0 to 10 centimeters. The range may mean the physical distance from portable apparatus 104 to the second apparatus 106. More precisely, the range may mean the physical distance of circuitries providing the proximity wireless connection for both of the apparatuses respectfully.

In an embodiment, the range of the proximity wireless connection 110 is between 0 to 1 meters.

In an embodiment, the range of the proximity wireless connection 110 is between 0 to 5 meters.

In an embodiment, the NFC is based on RFID technology.

In an embodiment, the NFC is provided by a NFC capable chip, such as RFID chip or a MIFARE™ chip.

In an embodiment, the NFC capable chip comprises an antenna.

In an embodiment, the NFC capable chip comprises a battery.

In an embodiment, the NFC capable chip comprises anti-tampering features.

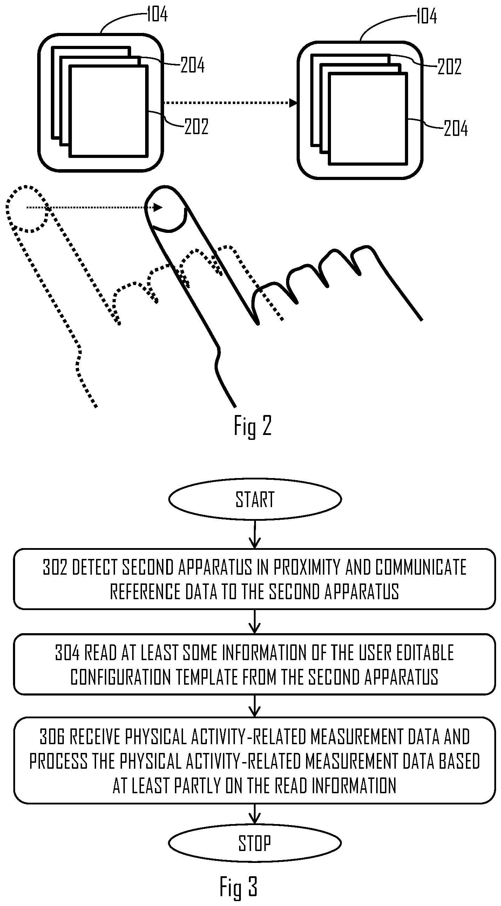

Figure 2 illustrates the selecting of data to be communicated via the proximity wireless connection. Referring to Figure 2, the portable apparatus 104 may comprise physical activity files 202, 204. In an embodiment, the portable apparatus 104 comprises reference data to physical activity files 202, 204 physically located, for example, in a network. The portable apparatus 104 may display the physical activity file 202 which dynamically configures the proximity wireless connection to communicate reference data associated with the physical activity file 202 to another apparatus as described previously. The user of the portable apparatus 104 may provide the device with user input, wherein the user input changes the displayed physical activity file 202 to the physical activity file 204. As the portable apparatus may, after the user input, display the physical activity file 204, the proximity wireless connection, such as proximity wireless connection 110 of the Figure 1A and Figure 1B, may be configured to communicate reference data associated with the physical activity file 204.

In an embodiment, the physical activity file comprises a training file.

In an embodiment, a specific training file is activated to be shown on the display of the portable apparatus 104.

In an embodiment, an index corresponding to the specific training file is communicated from the portable apparatus to the second apparatus 106.

In an embodiment, the second apparatus 106 receives the index corresponding to the specific training file.

In an embodiment, the index corresponding to the specific training file causes the second apparatus 106 to display the specific training file.

In an embodiment, the physical activity comprises physical exercise, such as walking, running, biking, weight lifting, white water sports, climbing, team sports to mention a few.

In an embodiment, the physical activity file comprises an exercise file.

In an embodiment, the physical activity comprises human daily normal activities such as sleeping, walking, sitting, standing to mention a few.

Figure 3 illustrates a flow diagram according to the invention. Referring to Figure 3, a portable apparatus, such as portable apparatus 104 of Figure 1A, Figure 1B and Figure 2, comprises a proximity wireless connection circuitry configured to detect a second apparatus, such as second apparatus 106 of Figure 1A and Figure 1B, in proximity to the portable apparatus and communicate reference data to the second apparatus, wherein the reference data causes the second apparatus to fetch and illustrate a user editable configuration template associated with the communicated reference data, as shown in block 302. The proximity wireless connection circuitry is further configured to read at least some information of the user editable configuration template from the second apparatus, as shown in block 304. The portable apparatus 104 further comprises a processing circuitry configured to receive physical activity-related measurement data and the read information of the user editable configuration template, and to process the physical activity-related measurement data, based at least partly on the read information of the user editable configuration template, to a physical activity-related parameter characterizing the physical activity.

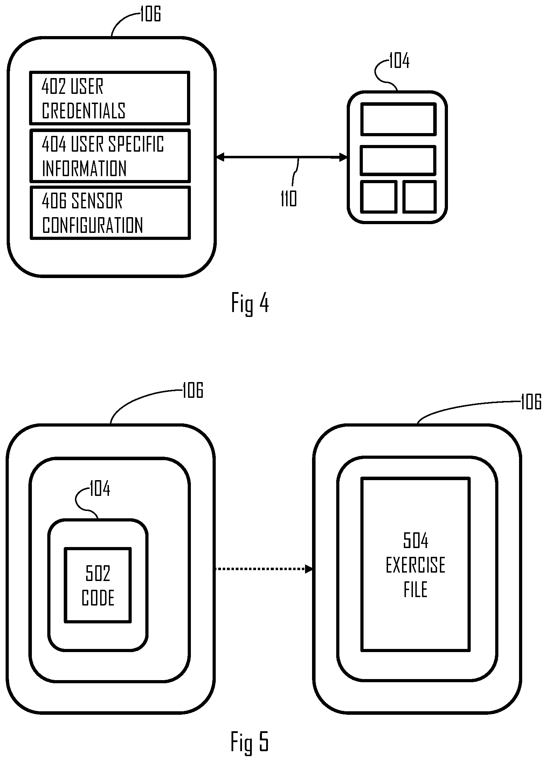

Figure 4 illustrates communication of information of the user editable configuration template. Referring to Figure 4, the proximity wireless connection 110 may be used to communicate information from the portable apparatus 104 to the second apparatus 106 and from the second apparatus 106 to the portable apparatus 104. The portable apparatus communicates reference data to the second apparatus 106 using the proximity wireless connection 110. The second apparatus may use the reference data to fetch data associated with the reference data from, for example, a database residing in a network. The fetched data may comprise a user editable configuration template comprising at least some of the following: user credentials 402, user specific information 404 and sensor configuration 406. The user may edit the information of the configuration template using the second apparatus 106. The user may, for example, provide the template user's height, weight and age. The user may change other personal settings, such as username and password, and further select the sensors to be used with the portable apparatus. In an embodiment, the selected sensors physically reside in the portable apparatus 104. In an embodiment, the information of the user editable configuration template is saved to the memory of the second apparatus 106 or to a database, such as database 112. The portable apparatus 104 reads the edited information of the user editable configuration template from the second apparatus 106 by using the proximity wireless connection 110. The portable apparatus 104 further uses the read information to configure the processing circuitry to process the physical activity-related measurement data based at least partly on the read information.

The information of the user editable configuration template is communicated to the portable apparatus 104 by the second apparatus 106.

The portable apparatus 104 receives the communicated data and performs an algorithm based on the received data.

In an embodiment, the read information of the user editable configuration template comprises at least one of the following: user credentials, user specific information and information of used sensors, as indicated in the Figure 4.

In an embodiment, the information of the user editable configuration template comprises data fields and corresponding field labels. The portable apparatus 104 may read only the data fields or some of the data fields of the user editable configuration template. This may reduce the amount of data to be transmitted and thus be more efficient. In an embodiment, the information of the user editable configuration template is composed as a single string parameter.

The user editable configuration template is a web-based template.

In an embodiment, the portable apparatus, such as portable apparatus 104, further comprises: a measurement circuitry configured to measure the physical activity-related measurement data of a user carrying out physical activity.

In an embodiment, the proximity wireless connection circuitry comprises a near field communication circuitry.

In an embodiment, the communicated reference data comprises a unique identifier. The unique identifier may offer a reliable means for identifying for example the user editable configuration template as it may not be mixed with other identifiers.

The communicated reference data comprises an address to a web location, and wherein the communicated reference data causes the second apparatus 106 to navigate to the said web location.

In an embodiment, the physical activity-related parameter comprises at least one of the following: heart activity information, energy-expenditure information, fitness-related information, activity information, and motion-related information.

In an embodiment, the physical activity-related measurement data is further processed, by the processing circuitry, on the basis of at least one of the following: user-related parameters describing characteristics of the user, physical activity-related parameters defining guidelines for a physical activity to be conducted, filtering parameters configuring filtering of the received physical activity-related measurement data.

In an embodiment, the processing circuitry is further configured to generate a physical activity file, such as physical activity files 202, 204 shown in Figure 2, comprising physical activity-related measurement data, and wherein the communicated reference data is associated with the physical activity file.

In an embodiment, the physical activity file comprises physical activity-related measurement data.

In an embodiment, the communicated reference data dynamically changes according to the physical activity file that is currently selected. Looking at the example of Figure 2, such a dynamical change of the communicated reference data may happen by changing the physical activity file 202 to physical activity file 204 by the user.

In an embodiment, the proximity wireless connection circuitry is further configured to transfer the physical activity file from the portable apparatus 104 to the training database 112.

In an embodiment, the communicated reference data, by the portable apparatus 104, causes the second apparatus 106 to fetch and illustrate the physical activity file.

In an embodiment, the proximity wireless connection circuitry is configured to automatically change the communicated reference data to correspond the physical activity file viewed by the user.

In an embodiment, the processing circuitry is further configured to cause sending of the physical activity file to the training database 112 and configured to determine a user selected physical activity file, and wherein the proximity wireless connection circuitry is further configured to communicate the reference data associated with the user selected physical activity file after the user selection to the second apparatus 106, wherein the communicated reference data enables the viewing of the user selected physical activity file on the second apparatus 106.

In an embodiment, the information comprised in the physical activity file is processed, by the processing circuitry, on the basis of at least one of the following: user-related parameters describing characteristics of the user, physical activity-related parameters defining guidelines for a physical activity to be conducted, filtering parameters configuring filtering of the received physical activity-related measurement data.

In an embodiment, the proximity wireless connection circuitry is further configured to transfer the physical activity file from the portable apparatus 104 to the training database 112.

In an embodiment, the communicated reference data, by the portable apparatus 104, causes the second apparatus 106 to fetch and illustrate the physical activity file. The physical activity file is stored in and fetched from the training database 112.

In an embodiment, the training database 112 is accessed via a web service.

In an embodiment, the apparatus further comprises at least one of the following sensors: a temperature sensor, a pressure sensor, heart activity sensor, optical heart activity sensor and a motion sensor.

In an embodiment, the proximity wireless connection circuitry comprises a dynamic near field communication circuitry with a reader capability.

The proximity wireless connection circuitry provides the portable apparatus 104 the proximity wireless connection 110.

The portable apparatus 104 receives reference data associated with the user editable configuration template from the second apparatus 106 via proximity wireless connection 110.

In an embodiment, the portable apparatus 104 fetches the user editable configuration template from the training database 112 or a web service, by using proximity wireless connection circuitry or other communication circuitry based on the received reference data.

The proximity wireless connection circuitry is configured to read the information of the user editable configuration template from the second apparatus.

In an embodiment, the processing circuitry is configured to run a physiological algorithm in the portable apparatus 104 on the basis of the user edited configuration template information.

In an embodiment, the second apparatus 106 uses NFC-reader to read portable apparatus' RFID-tag content emulating information which directs the second apparatus 106 to a web-based configuration tool. Settings may be fetched or set using the web-based interface on the second apparatus 106. The web-based interface may form, from the settings, a settings package which is loaded to the portable apparatus 104. The second apparatus 106 may configure the settings to the portable apparatus 104 by using the NFC connection.

In an embodiment, the web-based configuration tool is a mobile device application.

In an embodiment, the proximity wireless connection circuitry comprises a Quick Response (QR) circuitry.

Figure 5 illustrates communication of reference data according to an embodiment of the invention. Referring to Figure 5, the portable apparatus 104 may display a code 502 which is associated with a physical activity file or other portable apparatus data. The code may be, for example, a QR code. The second apparatus 106 may read the code 502 from the portable apparatus 104 by using, for example, a camera coupled with the second apparatus 106. The read code may cause the second apparatus 106 to open the physical activity file 504 from an external location indicated by the code and display the physical activity file on the second apparatus display. The external location may be a web address or a training database 112 address comprised in the read code.

In an embodiment, after a physical activity file has been transferred to a web service or a training database 112, the service or database returns a unique identifier of the physical activity file to the portable apparatus 104. This unique identifier may be added to a web address template and shown as a code, such as QR code, to a user. The code may be read by the user using an apparatus, such as mobile phone or a tablet computer, and the recorded physical activity file can be viewed quickly from the apparatus screen. The apparatus may be, for example, the second apparatus 106.

In an embodiment, the portable apparatus 104 is a training computer.

In an embodiment, the portable apparatus 104 is a physiological sensor.

In an embodiment, the various sensor devices 102 comprise a heart activity sensor.

In an embodiment, the various sensor devices 102 comprise an optical heart activity sensor.

In an embodiment, the various sensor devices 102 comprise a temperature sensor.

In an embodiment, the various sensor devices 102 comprise a pressure sensor.

In an embodiment, the various sensor devices 102 comprise a motion sensor.

In an embodiment, the second apparatus 106 is a mobile phone.

In an embodiment, the second apparatus 106 is a tablet computer.

In an embodiment, the second apparatus 106 is a training computer.

In an embodiment, the second apparatus 106 is a desktop computer.

In an embodiment, the second apparatus 106 is a laptop.

In an embodiment, the second apparatus is a television.

The second apparatus 106 comprises: a screen, an input unit, a connection circuitry and at least one processor and at least one memory including a computer program code, wherein the at least one memory and the computer program code are configured, with the at least one processor, to cause the second apparatus at least to: receive the communicated reference data from the portable apparatus 104 using the connection circuitry, display, on the screen, the user editable configuration template associated with the communicated reference data to the user, provide user edit means, by the input unit, for the user to edit the information of the user editable configuration template and communicate at least some information of the user editable configuration template to the portable apparatus 104.

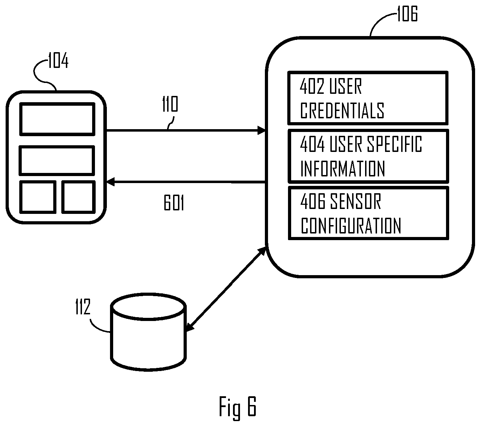

Figure 6 illustrates user editable configuration template transfer according to an embodiment of the invention. Referring to Figure 6, the portable apparatus 104 may detect the second apparatus 106 in proximity of the portable apparatus 104 and communicate reference data to the second apparatus 106 by using the proximity wireless connection 110. The communicated reference data causes the second apparatus to fetch user editable configuration template, associated with the communicated reference data, from a web location. In an embodiment, the user editable configuration template is fetched from a cloud service. The communicated reference data further causes the second apparatus 106 to display the user editable configuration template and allow the user of the second apparatus 106 to edit the information of the user editable configuration template. At least some of the information of the user editable configuration template is transferred to the portable apparatus 104 by using wireless communication 601.

In an embodiment, the wireless communication 601 is provided by a wireless communication circuitry.

In an embodiment, the wireless communication circuitry comprises a proximity wireless connection circuitry.

In an embodiment, the wireless communication 601 is provided by a Bluetooth capable circuitry.

In an embodiment, the information of the user editable configuration template comprises pre-settings for the portable apparatus 104.

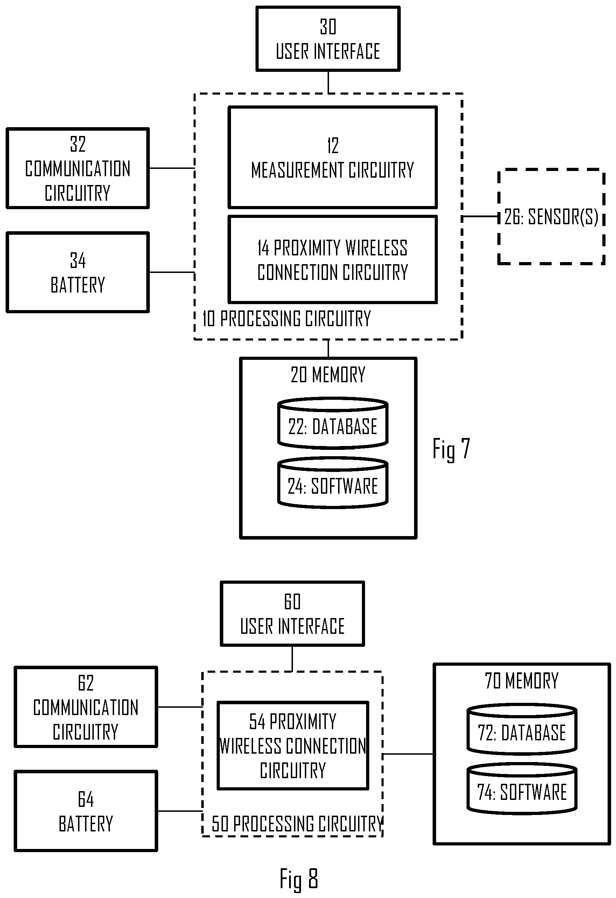

Figure 7 illustrates a block diagram of a portable apparatus according to an embodiment of the invention. The portable apparatus is comprised in the above-described portable apparatus 104. The portable apparatus may comprise a user interface 30. The user interface may comprise at least a display and an input device. The portable apparatus further comprises a communication circuitry 32 configured to provide the portable apparatus with wireless communication capability so that portable apparatus related data, such as physical activity files, may be transferred from the portable apparatus to another device, database or a network. The communication circuitry 32 is further configured to receive data, such as information of the user editable configuration template. The communication circuitry 32 may comprise well known wireless interface components such as one or more antennas, filters, amplifiers and frequency converters. The portable apparatus further comprises at least one of the various sensor devices 102. The internal sensor may be a heart rate sensor or a motion sensor, for example. The portable apparatus may further comprise the various sensor devices 102 of Figure 1A.

The apparatus further comprises a processing circuitry 10 which may comprise at least one processor(s). The processing circuitry 10 may comprise a measurement circuitry 12 configured to measure physical activity-related measurement data of a user carrying out a physical activity. The processing circuitry may receive the physical activity-related measurement data from the measurement circuitry 12 and process the physical activity-related measurement data. The processing circuitry 10 may, for example, a store physical activity file to the database 22 residing in a portable apparatus memory 20. The processing circuitry 10 further comprises a proximity wireless connection circuitry 14 which may be configured which may be configured to perform the functionalities of proximity wireless connection, for example proximity wireless connection 110 of Figure 1A, to detect a second apparatus in proximity to the portable apparatus and communicate reference data from the portable apparatus to the second apparatus, wherein the reference data is associated with a user editable configuration template, as described above. In an embodiment, the reference data is associated with a physical activity file. The proximity wireless connection circuitry is further configured to read at least some information of the user editable configuration template from the second apparatus.

The processing circuitry 10 is further configured to process the physical activity-related measurement data, based at least partly on the read information of the user editable configuration template, to a physical activity-related parameter characterizing the physical activity.

The memory 20 additionally stores one or more computer program products 24 defining the operation of the processing circuitry 10 and its circuitries 12 to 14. The circuitries 12 to 14 of the processing circuitry 10 may thus be considered as different computer program modules executed by the same physical circuitries of the processing circuitry 10.

The portable apparatus may further comprise a battery 34 for providing operating voltage for at least some of the above described circuitries, memory, sensors, such as various sensor devices 102, and user interface.

Figure 8 illustrates a block diagram of an apparatus according to an embodiment of the invention. The apparatus is comprised in the above-described second apparatus 106. The apparatus comprises a user interface 60. The user interface comprises at least a display and an input device. The apparatus further comprises a communication circuitry 62 configured to provide the apparatus with wireless communication capability so that portable apparatus -related data, such as user editable configuration template or a physical activity file, may be received from external location, such as web service. The communication circuitry 62 is further configured to communicate or transmit data, such as information of the user editable configuration template to the portable apparatus. The communication circuitry 62 may comprise well known wireless interface components such as one or more antennas, filters, amplifiers and frequency converters.

The apparatus further comprises a processing circuitry 50 which may comprise at least one processor(s). The processing circuitry 50 may comprise a proximity wireless connection circuitry 54 which may be configured to perform the functionalities of proximity wireless connection, for example proximity wireless connection 110 of Figure 1A, such as, receive reference data from the portable apparatus, as described above. The proximity wireless connection 54 may be further used to send data from the apparatus to the portable apparatus. The processing circuitry 50 is configured to use the received reference data to fetch and display the user editable configuration template which is associated with the received reference data. The processing circuitry 50 may additionally be configured to determine user input to the displayed user editable configuration template. The database 72 residing in memory 70 may be used to store the user editable configuration template. The processing circuitry 50 is configured to cause proximity wireless connection circuitry 54 to communicate the information of the user editable configuration template to the portable apparatus. The fetched, displayed, saved or edited data may be also the physical activity file.

The memory 70 may additionally store one or more computer program products 74 defining the operation of the processing circuitry 50 and circuitry 54. The circuitry 54 of the processing circuitry 50 may thus be considered as different computer program modules executed by the same physical circuitries of the processing circuitry 50.

The apparatus may further comprise a battery 64 for providing operating voltage for at least some of the above described circuitries, memory and user interface.

In an embodiment, the apparatuses of Figures 7 and 8 are realized as one apparatus, and a communication link may be provided between the apparatus of Figure 7 and the apparatus of Figure 8. The communication link may be wired or wireless.

As used in this application, the term 'circuitry' refers to all of the following: (a) hardware-only circuit implementations, such as implementations in only analog and/or digital circuitry, and (b) combinations of circuits and soft-ware (and/or firmware), such as (as applicable): (i) a combination of processor(s) or (ii) portions of processor(s)/software including digital signal processor(s), software, and memory(ies) that work together to cause an apparatus to perform various functions, and (c) circuits, such as a microprocessor(s) or a portion of a microprocessor(s), that require software or firmware for operation, even if the software or firmware is not physically present. This definition of 'circuitry' applies to all uses of this term in this application. As a further example, as used in this application, the term 'circuitry' would also cover an implementation of merely a processor (or multiple processors) or a portion of a processor and its (or their) accompanying software and/or firmware. The term 'circuitry' would also cover, for example and if applicable to the particular element, a baseband integrated circuit or applications processor integrated circuit for a mobile phone or a similar integrated circuit in a server, a cellular network device, or another network device.

The techniques and methods described herein may be implemented by various means. For example, these techniques may be implemented in hardware (one or more devices), firmware (one or more devices), software (one or more modules), or combinations thereof. For a hardware implementation, the apparatus(es) of embodiments may be implemented within one or more application-specific integrated circuits (ASICs), digital signal processors (DSPs), digital signal processing devices (DSPDs), programmable logic devices (PLDs), field programmable gate arrays (FPGAs), processors, controllers, micro-controllers, microprocessors, other electronic units designed to perform the functions described herein, or a combination thereof. For firmware or software, the implementation can be carried out through modules of at least one chip set (e.g. procedures, functions, and so on) that perform the functions described herein. The software codes may be stored in a memory unit and executed by processors. The memory unit may be implemented within the processor or externally to the processor. In the latter case, it can be communicatively coupled to the processor via various means, as is known in the art. Additionally, the components of the systems described herein may be rearranged and/or complemented by additional components in order to facilitate the achievements of the various aspects, described with regard thereto, and they are not limited to the precise configurations set forth in the given figures, as will be appreciated by one skilled in the art.

Embodiments as described may also be carried out in the form of a computer process defined by a computer program. The computer program may be in source code form, object code form, or in some intermediate form, and it may be stored in some sort of carrier, which may be any entity or device capable of carrying the program. For example, the computer program may be stored on a computer program distribution medium readable by a computer or a processor. The computer program medium may be, for example but not limited to, a record medium, computer memory, read-only memory, electrical carrier signal, telecommunications signal, and software distribution package, for example. Coding of software for carrying out the embodiments as shown and described is well within the scope of a person of ordinary skill in the art.

Even though the invention has been described above with reference to an example according to the accompanying drawings, it is clear that the invention is not restricted thereto but can be modified in several ways within the scope of the appended claims. Therefore, all words and expressions should be interpreted broadly and they are intended to illustrate, not to restrict, the embodiment. It will be obvious to a person skilled in the art that, as technology advances, the inventive concept, defined by the following claims, can be implemented in various ways.

## Claims

1. A portable apparatus (104) comprising:
sensor devices (102) for measuring and processing physical activity-related measurement data;
a proximity wireless connection circuitry (14) configured to detect a second apparatus (106) in proximity to the portable apparatus (104) and communicate reference data to the second apparatus (106),
wherein the reference data comprises an address to web location and causes the second apparatus (106) to fetch and illustrate a user editable configuration template from said web location, wherein the user editable configuration template is associated with the communicated reference data, wherein the user editable configuration template is a web-based template,
and wherein the proximity wireless connection circuitry (14) is further configured to read at least some information of the user editable configuration template from the second apparatus (106); and
a processing circuitry (10) configured to receive the physical activity-related measurement data and the read information of the user editable configuration template, to process the physical activity-related measurement data, based at least partly on the read information of the user editable configuration template, into a physical activity-related parameter characterizing the physical activity, and to perform an algorithm, based on the read information of the user editable configuration template, that configures sensor settings of the portable apparatus (104).

2. The apparatus of the claim 1, wherein the read information of the user editable configuration template comprises at least one of the following: user credentials (402), user specific information (404) and information of used sensors.

3. The apparatus of any preceding claim, wherein the proximity wireless connection circuitry (14) comprises a near field communication circuitry.

4. The apparatus of any preceding claim, wherein the communicated reference data comprises a unique identifier.

5. The apparatus of any preceding claim, wherein the configuring the sensor settings of the portable apparatus comprises selection of sensors to be used with the portable apparatus.

6. The apparatus of any preceding claim, wherein the physical activity-related parameter comprises at least one of the following: heart activity information, energy-expenditure information, fitness-related information, activity information, and motion-related information.

7. The apparatus of any preceding claim, wherein the physical activity-related measurement data is further processed, by the processing circuitry (10), on the basis of at least one of the following: user-related parameters describing characteristics of the user, physical activity-related parameters defining guidelines for a physical activity to be conducted, filtering parameters configuring filtering of the received physical activity-related measurement data.

8. The apparatus of any preceding claim, wherein the processing circuitry is further configured to generate a physical activity file (504) comprising physical activity-related measurement data, and wherein the communicated reference data is associated with the physical activity file (504).

9. The apparatus of the claim 8, wherein the communicated reference data dynamically changes according to the physical activity file (504) that is currently selected.

10. The apparatus of any of the claim 8 to 9, wherein the proximity wireless connection circuitry (140) is further configured to transfer the physical activity file (504) from the portable apparatus (104) to a training database (112).

11. The apparatus of any of the claim 8 to 10, wherein the communicated reference data, by the portable apparatus (104), causes the second apparatus (106) to fetch and illustrate the physical activity file.

12. A computer program product embodied on a distribution medium readable by a computer and comprising program instructions which, when loaded into a portable apparatus (106), execute a computer process comprising:
detect (302), using a proximity wireless connection circuitry (14), a second apparatus (106) in proximity to the portable apparatus (104) and communicate reference data to the second apparatus, wherein the reference data comprises an address to web location and causes the second apparatus (106) to fetch and illustrate a user editable configuration template from said web location, wherein the user editable configuration template is associated with the communicated reference data, wherein the user editable configuration template is a web-based template,
and wherein the proximity wireless connection circuitry (14) is further configured to read (304) at least some information of the user editable configuration template from the second apparatus (106);
receive (306) physical activity-related measurement data and process the physical activity-related measurement data, based at least partly on the read information of the user editable configuration template, into a physical activity-related parameter characterizing the physical activity; and
perform an algorithm, based on the read information of the user editable configuration template, that configures sensor settings of the portable apparatus (104).

13. A system comprising:
A portable apparatus (104) comprising:
sensor devices (102) for measuring and processing physical activity-related measurement data;
a proximity wireless connection circuitry (14) configured to detect a second apparatus (106) in proximity to the portable apparatus (104) and communicate reference data to the second apparatus (106), wherein the reference data comprises an address to web location and causes the second apparatus (106) to fetch and illustrate a user editable configuration template from said web location, wherein the user editable configuration template is associated with the communicated reference data, and wherein the proximity wireless connection circuitry (14) is further configured to read at least some information of the user editable configuration template from the second apparatus (106); and
a processing circuitry (10) configured to receive physical activity-related measurement data and the read information of the user editable configuration template, to process the physical activity-related measurement data, based at least partly on the read information of the user editable configuration template, into a physical activity-related parameter characterizing the physical activity, and to perform an algorithm, based on the read information of the user editable configuration template, that configures sensor settings of the portable apparatus (104);
the system further comprising: said second apparatus (106) comprising:
a screen;
an input unit;
a connection circuitry; and
at least one processor and at least one memory including a computer program code, wherein the at least one memory and the computer program code are configured, with the at least one processor, to cause the second apparatus at least to:
receive the communicated reference data from the portable apparatus (104) using the connection circuitry;
display, on the screen, the user editable configuration template associated with the communicated reference data to the user;
provide user edit means, by the input unit, for the user to edit the information of the user editable configuration template; and
communicate at least some information of the user editable configuration template to the portable apparatus.

## Patentansprüche

1. Tragbare Vorrichtung (104), umfassend:
Sensorvorrichtungen (102) zum Messen und Verarbeiten von auf körperliche Aktivität bezogene Messdaten;
eine drahtlose Näherungsverbindungsschaltung (14), die so konfiguriert ist, dass sie eine zweite Vorrichtung (106) in der Nähe der tragbaren Vorrichtung (104) erfasst und Referenzdaten an die zweite Vorrichtung (106) übermittelt,
wobei die Referenzdaten eine Adresse zu einem Web-Ort umfassen und veranlassen, dass die zweite Vorrichtung (106) eine vom Benutzer editierbare Konfigurationsvorlage von dem Web-Ort abruft und veranschaulicht, wobei die vom Benutzer editierbare Konfigurationsvorlage den kommunizierten Referenzdaten zugeordnet ist, wobei die vom Benutzer editierbare Konfigurationsvorlage eine webbasierte Vorlage ist,
und wobei die drahtlose Näherungsverbindungsschaltung (14) weiterhin dazu eingerichtet ist, wenigstens einige Informationen der von dem Benutzer editierbaren Konfigurationsvorlage aus der zweiten Vorrichtung (106) zu lesen; und
eine Verarbeitungsschaltung (10), die dazu eingerichtet ist, die mit der körperlichen Aktivität in Beziehung stehenden Messdaten und die gelesenen Informationen der vom Benutzer editierbaren Konfigurationsvorlage zu empfangen, um die auf physikalische Aktivität bezogenen Messdaten wenigstens teilweise basierend auf den gelesenen Informationen der vom Benutzer editierbaren Konfigurationsvorlage in einen auf körperliche Aktivität bezogenen Parameter zu verarbeiten, der die körperliche Aktivität charakterisiert, und einen Algorithmus basierend auf den gelesenen Informationen der vom Benutzer editierbaren Konfigurationsvorlage auszuführen, der die Sensoreinstellungen der tragbaren Vorrichtung (104) konfiguriert.

2. Vorrichtung nach Anspruch 1, bei der die gelesenen Informationen der vom Benutzer editierbaren Konfigurationsvorlage wenigstens eines der folgenden umfassen: Benutzeranmeldeberechtigungen (402), benutzerspezifische Informationen (404) und Informationen zu verwendeten Sensoren.

3. Vorrichtung nach einem der vorhergehenden Ansprüche, bei der die drahtlose Näherungsverbindungsschaltung (14) eine Nahfeldkommunikationsschaltung umfasst.

4. Vorrichtung nach einem der vorhergehenden Ansprüche, bei der die übermittelten Referenzdaten eine eindeutige Kennung aufweisen.

5. Vorrichtung nach einem der vorhergehenden Ansprüche, bei der das Konfigurieren der Sensoreinstellungen der tragbaren Vorrichtung eine Auswahl von Sensoren umfasst, die mit der tragbaren Vorrichtung verwendet werden sollen.

6. Vorrichtung nach einem der vorhergehenden Ansprüche, bei der der auf körperliche Aktivität bezogene Parameter wenigstens einen der folgenden Parameter umfasst: Herzaktivitätsinformationen, Energieverbrauchsinformationen, fitnessbezogene Informationen, Aktivitätsinformationen und bewegungsbezogene Informationen.

7. Vorrichtung nach einem der vorhergehenden Ansprüche, bei der die auf körperliche Aktivität bezogenen Messdaten von der Verarbeitungsschaltung (10) auf der Basis von wenigstens einem der folgenden Elemente weiterverarbeitet werden: benutzerbezogene Parameter, die Eigenschaften des Benutzers beschreiben; Parameter für körperliche Aktivität, die Richtlinien für eine auszuführende körperliche Aktivität definieren, Filterparameter zum Filtern der empfangenen Messdaten für körperliche Aktivität.

8. Vorrichtung nach einem der vorhergehenden Ansprüche, bei der die Verarbeitungsschaltung weiterhin dazu eingerichtet ist, eine physische Aktivitätsdatei (504) mit auf körperliche Aktivität bezogenen Messdaten zu erzeugen, und die kommunizierten Referenzdaten der physischen Aktivitätsdatei (504) zugeordnet sind.

9. Vorrichtung nach Anspruch 8, bei der sich die übermittelten Referenzdaten dynamisch gemäß der physischen Aktivitätsdatei (504) ändern, die aktuell ausgewählt ist.

10. Vorrichtung nach einem der Ansprüche 8 bis 9, bei der die drahtlose Näherungsverbindungsschaltung (140) weiterhin dazu eingerichtet ist, die Datei mit körperlicher Aktivität (504) von der tragbaren Vorrichtung (104) zu einer Trainingsdatenbank (112) zu übertragen.

11. Vorrichtung nach einem der Ansprüche 8 bis 10, bei der die mitgeteilten Referenzdaten durch die tragbare Vorrichtung (104) bewirken, dass die zweite Vorrichtung (106) die Datei mit körperlicher Aktivität abruft und veranschaulicht.

12. Computerprogrammprodukt, das auf einem von einem Computer lesbaren Verteilungsmedium ausgeführt ist und Programmanweisungen umfasst, die, wenn sie in ein tragbares Gerät (106) geladen werden, einen Computerprozess ausführen, der Folgendes umfasst:
Erfassen (302) unter Verwendung einer drahtlosen Näherungsverbindungsschaltung (14) einer zweiten Vorrichtung (106) in der Nähe der tragbaren Vorrichtung (104) und Übermitteln von Referenzdaten an die zweite Vorrichtung, wobei die Referenzdaten eine Adresse zum Web-Ort umfassen und bewirken, dass die zweite Vorrichtung (106) eine von dem Benutzer editierbare Konfigurationsvorlage von dem Web-Standort abruft und veranschaulicht, wobei die von dem Benutzer editierbare Konfigurationsvorlage den übermittelten Referenzdaten zugeordnet ist, wobei die vom Benutzer editierbare Konfigurationsvorlage eine webbasierte Vorlage ist,
und wobei die drahtlose Näherungsverbindungsschaltung (14) weiterhin dazu eingerichtet ist, wenigstens einige Informationen der von dem Benutzer editierbaren Konfigurationsvorlage aus der zweiten Vorrichtung (106) zu lesen (304);
Empfangen (306) von auf körperliche Aktivität bezogenen Messdaten und Verarbeiten der auf körperlicher Aktivität bezogenen Messdaten, die wenigstens teilweise auf den gelesenen Informationen der von einem Benutzer editierbaren Konfigurationsvorlage basieren, in einen auf körperliche Aktivität bezogenen Parameter, der die körperliche Aktivität charakterisiert; und
Ausführen eines Algorithmus' auf der Grundlage der gelesenen Informationen der vom Benutzer editierbaren Konfigurationsvorlage, der die Sensoreinstellungen des tragbaren Gerätes (104) konfiguriert.

13. System, umfassend:
eine tragbare Vorrichtung (104), umfassend:
Sensorvorrichtungen (102) zum Messen und Verarbeiten von auf körperliche Aktivität bezogenen Messdaten;
eine drahtlose Näherungsverbindungsschaltung (14), die dazu eingerichtet ist, eine zweite Vorrichtung (106) in der Nähe der tragbaren Vorrichtung (104) zu erfassen und Referenzdaten an die zweite Vorrichtung (106) zu übermitteln, wobei die Referenzdaten eine Adresse zum Web-Ort umfassen und die zweite Vorrichtung (106) veranlassen, eine vom Benutzer editierbare Konfigurationsvorlage von dem Web-Standort abzurufen und zu veranschaulichen, wobei die vom Benutzer editierbare Konfigurationsvorlage den übermittelten Referenzdaten zugeordnet ist und wobei die Näherungs-Funkverbindungsschaltung (14) weiterhin dazu eingerichtet ist, wenigstens einige Informationen der vom Benutzer editierbaren Konfigurationsvorlage von der zweiten Vorrichtung (106) zu lesen; und
eine Verarbeitungsschaltung (10), die zum Empfangen von auf physikalische Aktivitäten bezogenen Messdaten und der gelesenen Informationen der vom Benutzer editierbaren Konfigurationsvorlage eingerichtet ist, um die auf körperliche Aktivität bezogene Messdaten, die wenigstens teilweise auf den gelesenen Informationen der vom Benutzer editierbaren Konfigurationsvorlage basieren, in einen die körperliche Aktivität charakterisierenden körperbezogenen Parameter zu verarbeiten, und einen Algorithmus auf der Grundlage der gelesenen Informationen der vom Benutzer editierbaren Konfigurationsvorlage auszuführen, der Sensoreinstellungen des tragbaren Geräts (104) konfiguriert;
wobei das System weiterhin umfasst:
die zweite Vorrichtung (106) umfassend:
einen Bildschirm;
eine Eingabeeinheit;
eine Verbindungsschaltung; und
wenigstens einen Prozessor und wenigstens einen Speicher, der einen Computerprogrammcode enthält, wobei der wenigstens eine Speicher und der Computerprogrammcode mit dem wenigstens einen Prozessor so konfiguriert sind, dass sie die zweite Vorrichtung veranlassen zu dem:
Empfangen der übermittelten Referenzdaten von der tragbaren Vorrichtung (104) unter Verwendung der Verbindungsschaltung;
Anzeigen auf dem Bildschirm die von dem Benutzer editierbare Konfigurationsvorlage, die den mitgeteilten Referenzdaten für den Benutzer zugeordnet ist;
Bereitstellen von Benutzereditiereinrichtungen durch die Eingabeeinheit, damit der Benutzer die Informationen der vom Benutzer editierbaren Konfigurationsvorlage bearbeiten kann; und
Übermitteln wenigstens einiger Informationen der von dem Benutzer editierbaren Konfigurationsvorlage an das tragbare Gerät.

## Revendications

1. Appareil portable (104) comprenant :
des dispositifs capteurs (102) pour mesurer et traiter des données de mesure liée à une activité physique ;
un circuit de connexion sans fil par proximité (14) configuré pour détecter un deuxième appareil portable (106) à proximité de l'appareil portable (104) et communiquer des données de référence au deuxième appareil (106),
dans lequel les données de référence comprennent une adresse vers un emplacement Web et font que le deuxième appareil (106) récupère et illustre un modèle de configuration modifiable par un utilisateur à partir dudit emplacement Web, dans lequel le modèle de configuration modifiable par un utilisateur est associé à des données de référence communiquées, dans lequel le modèle de configuration modifiable par un utilisateur est un modèle fondé sur le Web,
et dans lequel le circuit de connexion sans fil par proximité (14) est en outre configuré pour lire au moins certaines informations du modèle de configuration modifiable par un utilisateur à partir du deuxième appareil (106) ; et
un circuit de traitement (10) configuré pour recevoir les données de mesure liées à une activité physique et les informations lues du modèle de configuration modifiable par un utilisateur, pour traiter les données de mesure liées à une activité physique, selon au moins partiellement les informations lues du modèle de configuration modifiable par un utilisateur, dans un paramètre lié à une activité physique caractérisant l'activité physique, et pour exécuter un algorithme, selon les informations lues du modèle de configuration modifiable par un utilisateur, qui configure des paramètres de capteur de l'appareil portable (104).

2. L'appareil de la revendication 1, dans lequel les informations lues du modèle de configuration modifiable par un utilisateur comprennent au moins un des suivants :
accréditations d'utilisateur (402), informations spécifiques d'utilisateur (404) et informations de capteurs utilisés.

3. L'appareil de l'une quelconque des revendications précédentes, dans lequel le circuit de connexion sans fil par proximité (14) comprend un circuit de communication en champ proche.

4. L'appareil de l'une quelconque des revendications précédentes, dans lequel les données de référence communiquées comprennent un identificateur unique.

5. L'appareil de l'une quelconque des revendications précédentes, dans lequel la configuration des paramètres de capteur de l'appareil portable comprend une sélection de capteurs à utiliser avec l'appareil portable.

6. L'appareil de l'une quelconque des revendications précédentes, dans lequel le paramètre lié à une activité physique comprend au moins un des suivants : informations d'activité cardiaque, informations de dépense énergétique, informations liées à la condition physique, informations d'activité et informations liées au mouvement.

7. L'appareil de l'une quelconque des revendications précédentes, dans lequel les données de mesure liées à une activité physique sont en outre traitées, par le circuit de traitement (10), selon au moins un des suivants : paramètres liés à un utilisateur décrivant des caractéristiques de l'utilisateur, paramètres liés à une activité physique définissant des directives pour une activité physique à mener, paramètres de filtrage configurant un filtrage des données de mesure liées à une activité physique reçues.

8. L'appareil de l'une quelconque des revendications précédentes, dans lequel le circuit de traitement est en outre configuré pour générer un fichier d'activité physique (504) comprenant des données de mesure liées à une activité physique, et dans lequel les données de référence communiquées sont associées au fichier d'activité physique (504).

9. L'appareil de la revendication 8, dans lequel les données de référence communiquées changent dynamiquement selon le fichier d'activité physique (504) qui est couramment sélectionné.

10. L'appareil de l'une quelconque des revendications 8 à 9, dans lequel le circuit de connexion sans fil par proximité (140) est configuré en outre pour transférer le fichier d'activité physique (504) de l'appareil portable (104) vers une base de données d'entraînement (112).

11. L'appareil de l'une quelconque des revendications 8 à 10, dans lequel les données de référence communiquées, par l'appareil portable (104), font en sorte que le deuxième appareil (106) récupère et illustre le fichier d'activité physique.

12. Produit de programme informatique incorporé dans un support de distribution lisible par un ordinateur et comprenant des instructions de programme qui, lorsque chargées dans un appareil portable (106), exécutent un processus informatique comprenant :
détecter (302), au moyen d'un circuit de connexion sans fil par proximité (14), un deuxième appareil (106) à proximité de l'appareil portable (104) et communiquer des données de référence au deuxième appareil, dans lequel les données de référence comprennent une adresse vers un emplacement Web et font que le deuxième appareil (106) récupère et illustre un modèle de configuration modifiable par un utilisateur à partir dudit emplacement Web, dans lequel le modèle de configuration modifiable par un utilisateur est associé aux données de référence communiquées, dans lequel le modèle de configuration modifiable par un utilisateur est un modèle fondé sur le Web,
et dans lequel le circuit de connexion sans fil par proximité (14) est en outre configuré pour lire (304) au moins certaines informations du modèle de configuration modifiable par un utilisateur à partir du deuxième appareil (106) ;
recevoir (306) des données de mesure liées à une activité physique et traiter les données de mesure liées à une activité physique, selon au moins partiellement les informations lues du modèle de configuration modifiable par un utilisateur, dans un paramètre lié à une activité physique caractérisant l'activité physique ; et
exécuter un algorithme, selon les informations lues du modèle de configuration modifiable par un utilisateur, qui configure des paramètres de capteur de l'appareil portable (104).

13. Système comprenant :
Un appareil portable (104) comprenant :
des dispositifs capteurs (102) pour mesurer et traiter des données de mesure liée à une activité physique ;
un circuit de connexion sans fil par proximité (14) configuré pour détecter un deuxième appareil (106) à proximité de l'appareil portable (104) et communiquer des données de référence au deuxième appareil (106), dans lequel les données de référence comprennent une adresse vers un emplacement Web et font que le deuxième appareil (106) récupère et illustre un modèle de configuration modifiable par un utilisateur à partir dudit emplacement Web, dans lequel le modèle de configuration modifiable par un utilisateur est associé aux données de référence communiquées, et dans lequel le circuit de connexion sans fil par proximité (14) est configuré en outre pour lire au moins certaines informations du modèle de configuration modifiable par un utilisateur à partir du deuxième appareil (106) ; et
un circuit de traitement (10) configuré pour recevoir des données de mesure liées à une activité physique et les informations lues du modèle de configuration modifiable par un utilisateur, pour traiter les données de mesure liées à une activité physique, selon au moins partiellement les informations lues du modèle de configuration modifiable par un utilisateur, dans un paramètre lié à une activité physique caractérisant l'activité physique, et pour exécuter un algorithme, selon les informations lues du modèle de configuration modifiable par un utilisateur, qui configure des paramètres de capteur de l'appareil portable (104) ;
le système comprenant en outre :
ledit deuxième appareil (106) comprenant :
un écran ;
une unité d'entrée ;
un circuit de connexion ; et
au moins un processeur et au moins une mémoire incluant un code de programme d'ordinateur, dans lequel l'au moins une mémoire et le code de programme d'ordinateur sont configurés, avec l'au moins un processeur, pour amener le dispositif à au moins :
recevoir les données de référence communiquées depuis l'appareil portable (104) en utilisant le circuit de connexion ;
afficher, à l'écran, le modèle de configuration modifiable par un utilisateur associé aux données de référence communiquées à l'utilisateur ;
fournir un moyen de modification par un utilisateur, par l'unité d'entrée, pour que l'utilisateur modifie les informations du modèle de configuration modifiable par l'utilisateur ; et
communiquer au moins certaines informations du modèle de configuration modifiable par un utilisateur à l'appareil portable.
